# FASCICULE DE BREVET EUROPEEN

(11) **EP 2 341 984 B1**
(45) Date de publication et mention de la délivrance du brevet: **08.04.2015**
(21) Numéro de dépôt: 09741403.1
(22) Date de dépôt: 11.09.2009
(51) Int. Cl.: A61N 5/06, A61B 18/18, A61B 18/20, A61B 18/22

(54) **PIECE A MAIN A CARTOUCHE POUR APPAREIL DE PHOTOTRAITEMENT DE LA PEAU**
HANDSTÜCK MIT KARTUSCHE FÜR EIN GERÄT ZUR LICHTBEHANDLUNG DER HAUT
HANDPIECE WITH CARTRIDGE FOR A SKIN PHOTO-TREATMENT APPARATUS

(30) Priorité: 16.09.2008 FR 0856204; 09.09.2009 FR 0956126
(43) Date de publication de la demande: 13.07.2011
(73) Titulaire: Dermeo, 75018 Paris (FR)
(72) Inventeur: HOTTINGER, Christophe, F-75009 Paris (FR); TANNOUS, Pascale, F-75009 Paris (FR)
(74) Mandataire: Cabinet HERRBURGER
(86) Numéro de dépôt international: PCT/FR2009/051712
(87) Numéro de publication internationale: WO 2010/031947

(56) Documents cités:
- EP-A- 1 535 582
- WO-A-03/043514
- WO-A-2007/007167
- WO-A-2008/012519
- WO-A-2008/070747
- WO-A-2008/088792
- WO-A-2008/088795
- US-A1- 2007 198 004

## Description

### Domaine de l'invention

La présente invention concerne une pièce à main pour un appareil de phototraitement cosmétique, médical/thérapeutique de la peau, reliée à un poste d'alimentation en énergie et en fluide caloporteur de refroidissement par une liaison pas câble et tubes dans l'appareil et, comprenant
- une coque logeant, de manière amovible, une cartouche à fenêtre ayant une source lumineuse et son circuit de refroidissement et un capot fermant l'emplacement de la cartouche dans la coque,
- un guide de lumière relié à la source lumineuse par la fenêtre de la cartouche et dont la surface de sortie est destinée à être appliquée contre ou au-dessus de la surface de la peau à traiter.

### Etat de la technique

On connaît différents types de pièces à main pour de tels appareils. Ces pièces à main logent la source de lumière commandée ainsi que le guide de lumière transmettant la lumière de la source vers la surface de sortie de la pièce à main qui vient au-dessus ou en contact de la peau, par exemple selon le document WO 02/08 2866.

Il est également connu selon le document EP 1 535 582 de réaliser une pièce à main pour un appareil de phototraitement cosmétique, médical/thérapeutique de la peau ayant une partie amovible, fixée par vissage. Cette partie amovible ne se compose pas seulement de la cartouche mais de l'ensemble optique en aval de celle-ci. Il s'agit d'un boîtier avec une fenêtre de sortie et une pièce destinée à éviter le contact direct entre le filtre et la peau. Cet ensemble coulisse dans des rails dans une ouverture réalisée dans le dessous de la poignée. Mais dans ce document, il n'y a pas d'embase au-dessus du guide de lumière et des organes de connexion de la cartouche qui seraient reliés à l'embase puisque l'ensemble se glisse dans la poignée.

Selon le document WO 2008/088792, on réalise une pièce à main dont la sortie optique est constituée par une brosse à fibres optiques de manière à canaliser le flux lumineux sur les surfaces ponctuelles à la sortie des fibres optiques.

La partie composée du faisceau de fibres optiques et du support guidant les fibres vers des tiges constituant des guides de lumière et qui sont parallèles pour fournir des points lumineux, est détachable. Mais il ne s'agit pas d'une cartouche au sens de la présente invention ni de l'amovibilité de la partie optique de l'appareil dont la structure est totalement différente de celle de la présente invention, cette partie optique se composant notamment d'un miroir de renvoi et d'une lentille de focalisation pour regrouper le faisceau lumineux sur la surface d'entrée constituée par le regroupement des extrémités des fibres optiques.

Selon le document WO 2008/070747, on connaît un autre type de pièce à main pour un appareil de phototraitement de la peau. Cet appareil comporte différentes têtes optiques mais rien n'est dit sur l'interchangeabilité de la cartouche constituant la source lumineuse.

Il est également connu selon le document WO 03/043514, de réaliser une pièce à main d'appareil dont toute la partie optique peut être enlevée de la pièce à main. Cette partie optique constitue un bloc. Il ne s'agit pas de la lampe émettant les flashs mais de l'ensemble de l'équipement d'alimentation et de commande de fonctionnement de cette lampe.

Il existe également selon le document WO 2008/012519, une cartouche constituant une source de lumière pulsée munie de moyens de connexion électriques et hydrauliques pour l'alimentation de la lampe et son refroidissement. Mais il s'agit d'une simple connexion par enfichage sans aucun moyen de guidage et de positionnement autre pour la cartouche comportant la lampe.

Une cartouche pour une pièce à main est connue selon le document WO 07/007167.

L'inconvénient de cette cartouche est sa tenue et sa liaison tant électrique que fluidique avec les éléments complémentaires de la pièce à main. Cela crée des problèmes de fuites ou de contacts électriques réduisant la durée de vie de la lampe.

De façon plus détaillée, la connectique de la liaison de la cartouche est insuffisante ; elle risque de se déconnecter sous l'effet des flashs qui créent des vibrations. Ces vibrations créent aussi des risques de fuites et de ruptures des connections électriques.

La cartouche est mal tenue de sorte qu'elle ne peut fournir correctement l'énergie lumineuse à cause du mauvais contact avec le guide de lumière. Cette mauvaise tenue risque également d'endommager les connecteurs.

Cette cartouche nécessite une sonde de température pour éviter la montée en température au-delà d'un seuil critique pour éviter de blesser le patient par une montée en température du matériel, trop élevée, qui risquerait de le brûler.

### But de l'invention

La présente invention a pour but de développer une pièce à main pour un appareil de phototraitement cosmétique, médical/thérapeutique de la peau, permettant de remplacer facilement la cartouche contenant la source lumineuse, assurant une meilleure étanchéité pour la liaison fluidique et un meilleur contact pour améliorer la durée de vie de la cartouche et faciliter la mise en place et son utilisation en évitant les arrêts de fonctionnement liés à une éventuelle surchauffe.

### Exposé et avantageas de l'invention

A cet effet, l'invention concerne une pièce à main du type défini ci-dessus caractérisé en ce que
- l'emplacement de la cartouche au-dessus du guide de lumière, est défini par une embase portant les organes de connexion de la cartouche et par une surface de guidage dont une extrémité correspond à l'embase et les côtés sont des rails latéraux de guidage autour de l'emplacement du guide de lumière et
- la cartouche est en forme de boîtier plat dont une extrémité porte une plaque transversale d'assemblage munie des organes de connexion homologues à ceux de l'embase et les côtés comportent des nervures de guidage pour coopérer avec les rails latéraux de l'emplacement, positionner la cartouche et la guider dans son mouvement de translation vers son emplacement pour le branchement et la connexion en fin de course et la maintenir connectée.

La cartouche de cette pièce à main est facilement accessible et son démontage se fait par un simple mouvement de translation. La cartouche est néanmoins tenue fermement dans son emplacement grâce aux rails de guidage qui maintiennent la cartouche appliquée contre son emplacement et contre l'embase évitant tout risque de déconnection.

Suivant une autre caractéristique avantageuse,
- à l'emplacement de la cartouche, la coque comporte des rails supplémentaires parallèles à ceux destinés à guider la cartouche, ainsi qu'un organe d'enclipsage,
- le capot couvrant l'emplacement de la cartouche et complétant la coque, comporte des organes de guidage pour coulisser dans les rails supplémentaires, ainsi qu'un organe d'enclipsage complémentaire pour coopérer avec celui de la coque en position de fermeture du capot.

Le capot couvrant l'emplacement de la cartouche et qui se met en place par coulissement puis verrouillage par un organe d'enclipsage, bloque le capot et par suite, complète le maintien de la cartouche dans son emplacement.

De façon avantageuse, le capot comporte intérieurement une butée souple pour s'appuyer contre l'extrémité avant de la cartouche et compléter son maintien déjà assuré par les nervures et les rails et l'engagement des organes de connexion dans l'embase. La cartouche est ainsi parfaitement maintenue à son emplacement pour une bonne transmission du flux lumineux injecté par la source lumineuse dans le guide de lumière.

Suivant une autre caractéristique, le boîtier de la cartouche comporte deux zones latérales de préhension pour permettre de pincer le boîtier à la main ou le dégager et le mettre en place, par un mouvement de coulissement de ses nervures dans les rails.

Cette forme particulière du boîtier facilite l'extraction de la cartouche ou sa mise en place, sans nécessiter de pincement trop vigoureux de la cartouche.

Enfin, ces deux zones latérales de préhension constituent des surfaces de préhension incitant l'utilisateur à y tenir la cartouche pour éviter tout contact des doigts avec le fenêtre de la source lumineuse de la cartouche. Cela est particulièrement important lorsque la cartouche est prise en dehors de la coque et se trouve dans une orientation quelconque.

Dans le cas de la cartouche insérée dans l'embase de la coque, la fenêtre de la cartouche est naturellement tournée vers le guide de lumière alors que la main vient contre l'autre face de la cartouche pour la saisir par les deux zones latérales de préhension.

Suivant une autre caractéristique avantageuse,
- le boîtier de la cartouche est formé de deux parties logeant la source lumineuse et son circuit de fluide caloporteur de refroidissement,
- l'une des parties a une fenêtre dans laquelle apparaît la source lumineuse pour transmettre son flux lumineux,
- les deux parties étant assemblées selon un plan de jonction sensiblement parallèle au plan de l'emplacement,
- la partie inférieure porte les nervures et son extrémité, porte la plaque d'assemblage.

Le boîtier présente une forme simple à réaliser malgré la complexité des éléments constituant le boîtier de la cartouche et, en particulier, le circuit de liquide caloporteur de refroidissement.

La coque est réalisée d'une façon avantageuse en ce qu'elle est composée de deux parties se rejoignant sensiblement dans le plan médian et du capot au-dessus de l'emplacement de la cartouche, ainsi que de logements pour recevoir l'embase pour le branchement de la cartouche.

De façon avantageuse, le capot comporte un ventilateur sous sa paroi supérieure munie d'orifices de ventilation et au-dessus de l'emplacement de la cartouche,
- la coque ayant dans ses parois latérales, de part et d'autre de la cartouche, des fentes de ventilation pour la sortie d'air,
- un chemin de passage d'air étant formé entre les orifices du dessus du capot, à travers le ventilateur et le dessus de la cartouche pour se diviser vers les côtés de la cartouche et sortir par les fentes de ventilation de la coque à côté de la cartouche.

La ventilation forcée de l'enceinte de la poignée, délimitée par la coque et le capot enfermant la cartouche, évite la surchauffe de cette enceinte et les arrêts de fonctionnement intempestifs, qui nécessiteraient un arrêt plus ou moins prolongé de l'appareil.

L'intégration du ventilateur dans la pièce à main est particulièrement simple du fait que le ventilateur est associé au capot et non à la coque. Cela simplifie la fabrication des moules servant à la réalisation de la pièce à main.

Suivant une caractéristique avantageuse, le capot comporte deux broches d'alimentation électrique du moteur du ventilateur pour venir dans des douilles de la coque à côté du crochet de verrouillage.

Cette façon d'alimenter le ventilateur du capot facilite les interventions à l'intérieur de l'enceinte, c'est-à-dire par exemple le remplacement de la cartouche sans avoir à démonter l'alimentation du ventilateur puisque le branchement se fait automatiquement par la mise en place du capot, d'autant plus que, selon l'invention, le capot coulisse dans un rail de la coque pour venir en position d'assemblage, la phase finale étant réalisée par l'accrochage du crochet du capot dans un logement correspondant de la coque.

Suivant une autre caractéristique, un circuit de commande pour le fonctionnement du ventilateur (vitesse de fonctionnement/débit) relié à une sonde de température associée à l'enceinte de la poignée (coque et capot) pour commander le fonctionnement de ventilateur en fonction de la température détectée à l'intérieur de l'enceinte de la pièce à main.

Ce mode de fonctionnement est adapté à l'intensité de l'utilisation de la pièce à main, évitant de faire tourner le moteur du ventilateur de manière excessive.

En outre, par rapport à l'état de la technique, comme la circulation forcée d'air, arrive sur le dessus de la cartouche, à l'endroit le plus chaud de celle-ci puisque situé au dos de la lampe à décharge, on n'atteint jamais une température provoquant l'arrêt de fonctionnement de la machine. Ainsi, on évite le temps de récupération qui selon l'état de la technique, peut être très long car en cas de dépassement de la température autorisée pour le fonctionnement, la machine s'arrête et ne peut recommencer à fonctionner dès que l'on passe en dessous de cette limite. Or, la situation est alors précaire puisque très rapidement la température redépasse la limite et l'appareil s'arrête de nouveau. Ce fonctionnement intermittent des installations connues est totalement évité par la circulation forcée d'air et le refroidissement qui en résulte de l'enceinte de la pièce à main.

Suivant une autre caractéristique avantageuse, le boîtier de ventilateur est clipsé dans des languettes de retenue moulées en une seule pièce avec le capot.

Ce mode de réalisation est intéressant pour le moulage de la pièce, c'est-à-dire du capot ; la mise en place du ventilateur se fait dans des conditions particulièrement simples. Après cette mise en place, il suffit de souder les deux fils d'alimentation aux broches intégrées dans le capot de la pièce à main, par exemple au moment de l'injection de la pièce à main.

### Dessins

La présente invention sera décrite ci-après de manière plus détaillée à l'aide d'un exemple de réalisation d'une pièce à main représentée schématiquement dans les dessins annexés dans lesquels :
- la figure 1 est une vue isométrique d'un mode de réalisation de la pièce à main,
- les figures 2, 3 montrent respectivement une vue de côté et une vue de dessus de la pièce à main de la figure 1,
- la figure 4 est une vue en coupe par un plan médian de la pièce à main de la figure 1,
- les figures 5A-5D sont des vues en coupe selon les plans de coupe A, B, C, D de la figure 4,
- la figure 6 est une vue en coupe selon le plan VI VI de la figure 3,
- la figure 7 est une vue en coupe selon le plan BB de la figure 4 à échelle agrandie et sans la cartouche,
- la figure 8 est une vue isométrique du capot de la coque,
- la figure 9 est une vue isométrique de la cartouche et de l'embase alignées en vue de leur connexion,
- la figure 10A est une vue de dessus de la cartouche de la figure 9,
- la figure 10B est une vue de côté de la cartouche de la figure 9,
- la figure 10C est une vue de dessous de la cartouche de la figure 9,
- la figure 11 est une vue en perspective d'une variante de réalisation d'une pièce à main selon l'invention,
- la figure 12 est une vue en perspective du capot de la pièce à main de la figure 11,
- la figure 13 est une vue en coupe longitudinale partielle montrant la partie modifiée de la pièce à main selon cette variante,
- la figure 14 est une vue en perspective de dessous du capot de la pièce à main.

### Description d'un mode de réalisation

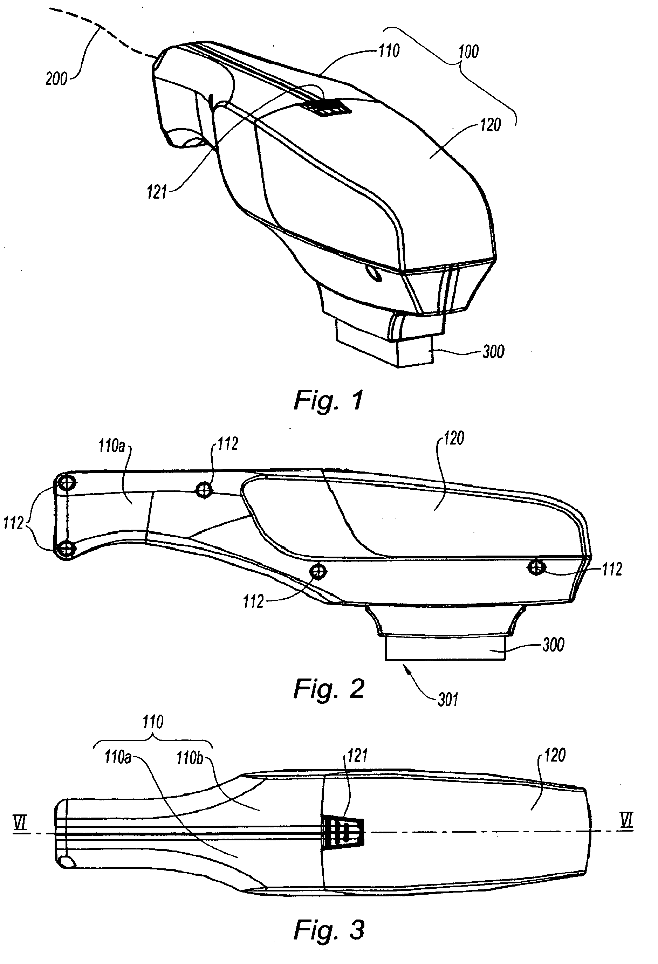

Selon les figures 1, 2 et 3, l'invention concerne une pièce à main pour un appareil de phototraitement cosmétique, médical ou thérapeutique de la peau. Cette pièce à main 100 est reliée par des liaisons fluidiques et électriques (tuyaux, câbles) 200 à un poste d'alimentation en énergie et en fluide caloporteur de refroidissement. Les tubes et les câbles de liaison ne sont pas détaillés.

La pièce 100 a une coque 110 formant une poignée reliée aux tubes et câbles 200 et une tête pour le phototraitement ; la coque 110 loge une cartouche équipée d'une source lumineuse commandée pour émettre une lumière de longueur d'onde donnée et suivant une émission programmée et commandée, par exemple une émission impulsionnelle, une émission de flashs, une succession d'impulsions selon une fréquence et une suite d'impulsions commandées.

La lumière est émise par la pièce à main par l'intermédiaire d'un guide de lumière 300 reliant la source lumineuse à l'extérieur, en formant une surface d'émission 301 destinée à venir contre la zone de la peau à traiter ou au-dessus de celle-ci, en fonction de la nature du traitement à effectuer. Le guide de lumière 300 a, le cas échéant, également, une fonction de filtre pour ne laisser passer qu'un rayonnement de longueur d'onde précise.

Selon la figure 3, la coque 110 est composée de deux parties 110a, 110b sensiblement symétriques qui se rejoignent selon le plan médian vertical VI VI de la pièce à main (par convention, cette direction du plan vertical est celle de la feuille du dessin de la figure 2) en laissant sur le dessus, une zone recevant un capot 120 et correspondant au moins en partie à l'emplacement de la cartouche. Les deux parties 110a, b de la coque 110 sont assemblées par l'intermédiaire de liaisons vissées ou points de vissage 112 transversales et le capot 120 s'installe sur l'ouverture par coulissement et se verrouille en position fermée. Le verrouillage est fait par un crochet intégré au capot 120 qui se libère en agissant sur un poussoir 121 apparaissant sur le dessus du capot.

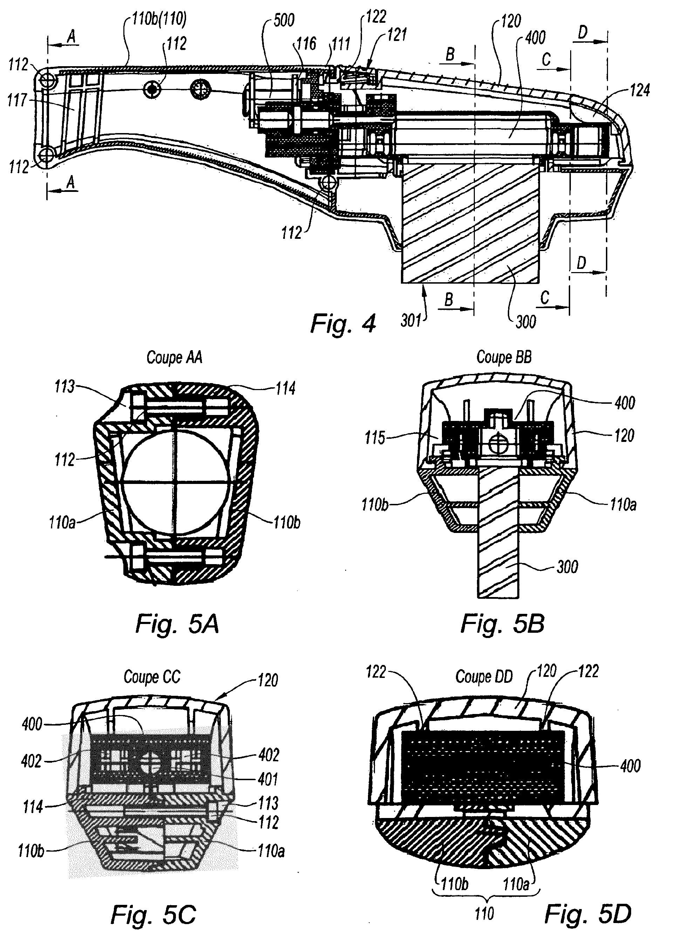

La vue en coupe de la figure 4 par le plan de symétrie, montre plus particulièrement la structure de la coque et de l'emplacement de la cartouche 400 ainsi que du guide de lumière 300. Cette vue de la demie-coque 110b selon la plan de symétrie VI VI est une coupe montrant les deux parties de la coque qui s'imbriquent suivant leur plan de jonction par une liaison par chevauchement des bords, le cas échéant de type rainure et languette. Le crochet 122 combiné au poussoir 121 est articulé au couvercle pour venir dans un organe d'accrochage 111 dans la position homologue, sur le côté intérieur de la coque. Les deux parties de la coque 110 sont vissées l'une à l'autre par les vis 112 sensiblement perpendiculaires au plan médian VI VI comme cela apparaît aux figures 5A, 5C. Pour les points de vissage 112, les parties 110a, b de la coque 110 comportent alternativement l'une, des orifices renforcés 113 pour le passage des vis 112 et l'autre, des cheminées de vissage 114 dans lesquelles les vis 112 se taillent leur logement ou qui intègrent des écrous surmoulés dans la matière de la coque. Les points de vissage sont répartis à l'extrémité de la poignée, au milieu de celle-ci et au niveau de l'emplacement de la cartouche 400 et du guide de lumière 300, sous le capot 120. L'emplacement de la cartouche situé au-dessus du guide de lumière 300 est réalisé comme le montrent les figures 4, 5A, B, C, D, 6, 7.

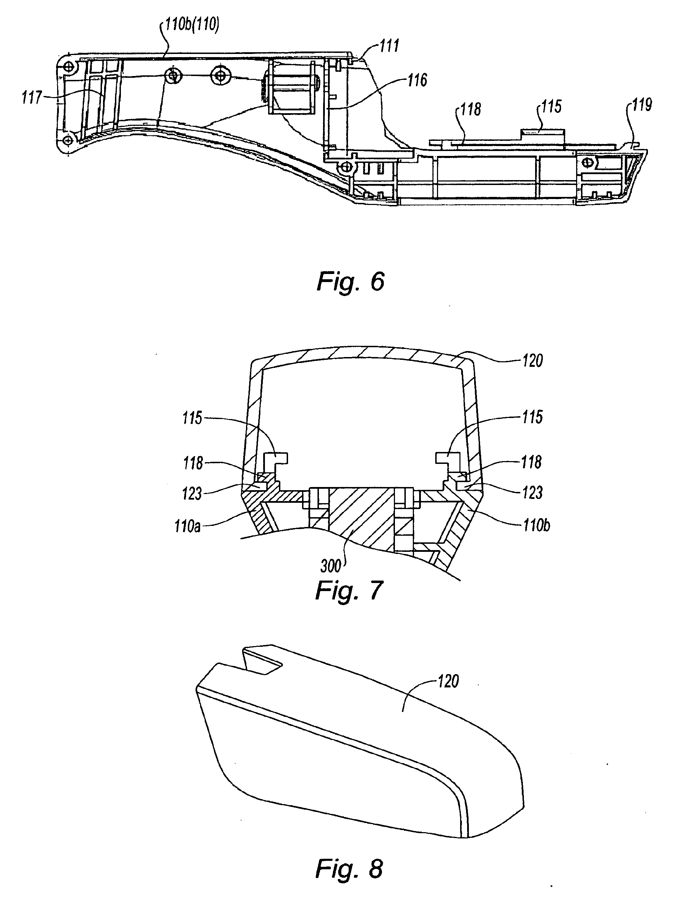

Selon la figure 4, l'emplacement de la cartouche 400 située au-dessus du guide de lumière 300 est formé à la fois par des glissières latérales 115 (figures 6 et 7) dans les deux parties 110a, b de la coque 110 et en extrémité, du côté de la poignée, par une embase 500 encastrée par une liaison par la forme, éventuellement complétée par vissage, dans des logements dans les deux parties 110a, b de la coque 110 au moment de leur assemblage. Pour cela, les deux parties 110a, b comportent des nervures 116. L'embase 500 est elle-même reliée aux tuyaux d'arrivé/sortie de fluide caloporteur et au câble électrique d'alimentation de la source lumineuse et le cas échéant à un câble de transmission de signaux, alimentant une surface d'affichage telle qu'un écran. Cette surface d'affichage (non représentée) prévue sur le dessus de la pièce à main, affiche par exemple l'état de fonctionnement et des signaux de capteurs intégrés à la pièce à main tels qu'un capteur de température, un capteur de contact ou un photocapteur pour détecter la température de fonctionnement, la présence du guide de lumière 300 ou la présence et/ou la nature de la surface placée devant le guide de lumière. L'affichage sur la poignée indique le programme choisi et autres caractéristiques reprenant, pour des raisons de convenance, au moins en partie l'affichage fait sur le poste central.

Les informations à échanger entre la poignée et l'appareil peuvent également être des signaux fournis par des capteurs non représentés, intégrés dans la pièce à main tels que des capteurs de température asservissant le circuit de fluide caloporteur ou des circuits de sécurité ou de connexion de la cartouche 400.

La vue en coupe de la figure 4 et la vue de côté de la demi-coque 110b de la figure 6 montrent plus particulièrement l'emplacement de la cartouche et les rails latéraux 115 pour glisser la cartouche dans son emplacement, par un mouvement de droite vers la gauche selon l'orientation de la figure 6, jusqu'à ce que les éléments de connexion et de branchement de la cartouche 400 soit branchés dans ceux de l'embase 500.

La vue de côté de la demi-coque 110b selon la figure 6 montre les nervures de renforcement 116 pour la fixation de l'embase 500 et les nervures 117 formant un serre-câble à la sortie de la poignée.

Les figures 5A-5D montrent plus particulièrement la structure de la coque dans différents plans de coupe transversale.

La figure 5A est une vue en coupe au niveau des points de vissage à l'extrémité de la poignée. Cette vue laisse apparaître les vis 112 logées dans les cheminées de vissage 114 de la partie 110b en ayant traversé l'orifice renforcé 113 de la partie de coque 110a.

La figure 5B montre la coupe de la cartouche 400 au-dessus du guide optique 300 avec les rails 115 de la coque 110 coopérant avec le boîtier de la cartouche 400.

La figure 5C est une vue en coupe de l'avant de la cartouche 400, laissant apparaître le logement central de la source lumineuse 401 bordée de chaque côté par le canal 402 de fluide caloporteur.

Au niveau de cette coupe, il y a également une liaison vissée avec une vis 112 logée dans une cheminée à vis 114 en traversant un orifice renforcé 113 dans chacune des parties 110a, 110b de la coque.

Enfin, la figure 5D est une vue en coupe de l'extrémité avant de la coque 110 et du capot 120, au niveau des éléments d'appui 122 en forme de nervures du capot 120.

Le capot 120 apparaissant aux figures 7 et 8 a une forme complémentaire de celle de l'ouverture autour de l'emplacement de la cartouche, formé par l'assemblage des deux moitiés de la coque. Le capot 120 présente un bord inférieur avec un retour 123 pour coopérer avec les rails latéraux supplémentaires 118 situés à la base de l'emplacement de la cartouche, de chaque côté. Ainsi et comme le montre la figure 3, le capot 120 mis en place par un mouvement de coulissement, bloque la cartouche 400 par sa butée 124. A l'avant le capot 120 est retenu par un bec 119 de la coque 110 et à l'arrière, par son crochet 122 dans l'organe d'accrochage 111. Comme le capot 120 est ainsi encastré et verrouillé, la cartouche 400 est ainsi verrouillée. Pour dégager la cartouche 400, il faut d'abord déverrouiller le capot 120 puis le glisser pour le dégager. La cartouche 400 peut alors être dégagée de son emplacement pour être remplacée par une cartouche ayant d'autres caractéristiques optiques.

La figure 8 montre la forme du capot 120.

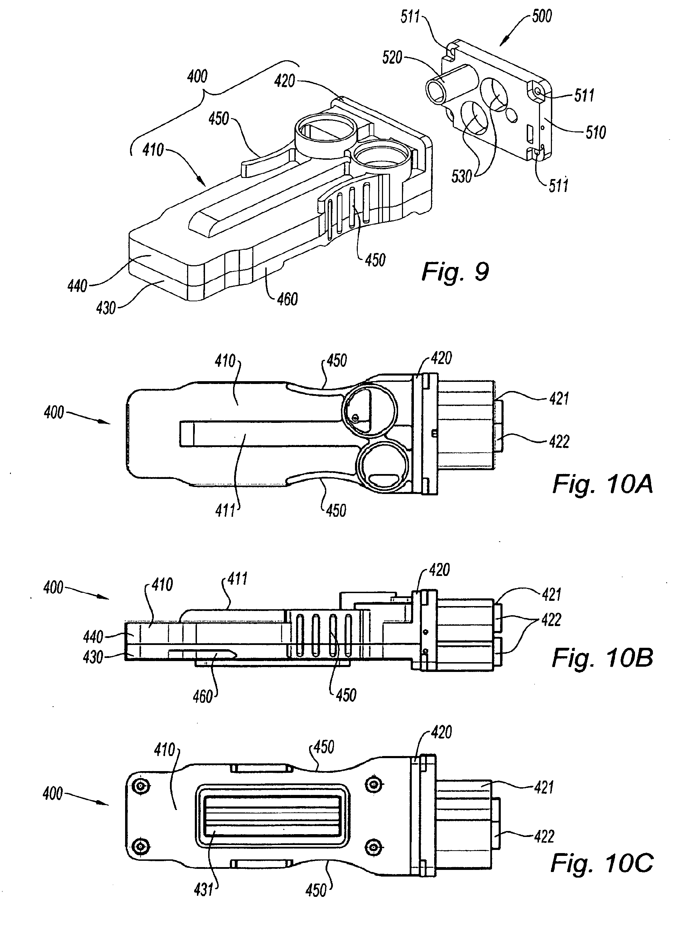

L'embase 500 et la cartouche 400 apparaissent de façon plus détaillée aux figures 9 et 10A, B, C.

Selon la figure 9, l'embase 500 est constituée par une plaque 510 qui se glisse dans le logement transversal formé dans chacune des moitiés 110a, b de la coque 110 par des nervures. Le logement est constitué lorsque la coque est assemblée. La plaque 510 de l'embase comporte des orifices de vissage 511 permettant en plus de visser l'embase sur les deux parties de la coque.

La face avant de l'embase 500 est munie d'un connecteur pour les branchements électroniques/électriques 520 et deux ajutages 530 pour recevoir des embouts de raccordement du circuit de fluide caloporteur de la cartouche 400.

La cartouche 400 présentée en position alignée sur l'embase 500 pour y être engagée, est constituée par un boîtier allongé 410, aplati, terminé d'un côté par une plaque d'assemblage 420 destinée à être réunie à l'embase 500. La plaque d'assemblage 420 comporte des éléments mâle/femelle 421 correspondant à ceux 520, 530 de l'embase 500, à savoir : une prise ou connecteur 421 complémentaire destiné à coopérer avec le connecteur 520 de l'embase et deux embouts 422 avec des joints qui s'introduisent dans les deux ajutages 530 de branchement de fluide caloporteur.

La cartouche 400 est formée d'un boîtier en deux parties, une partie inférieure 430 et une partie supérieure 440. Une fois assemblées, ces deux parties ont un contour commun qui comprend deux zones de préhension 450 de forme incurvée, munies de nervures ou d'aspérités pour favoriser la tenue de la cartouche 400 et sa manoeuvre d'extraction ou d'enfoncement dans l'embase 500. Les deux zones de préhension 450 ont une hauteur importante, dépassant de la forme de base du boîtier 410 pour constituer des surfaces de préhension, offrant une bonne prise aux doigts.

Les côtés du boîtier de la cartouche comportent, au niveau de la partie inférieure 430, deux nervures de guidage 460 destinées à coopérer avec les rails latéraux 115 de la coque 110.

L'embase 500 ou la coque peuvent également comporter un moyen de détection pour détecter le type de cartouche utilisée et n'autoriser que les programmes de fonctionnement appliqués par l'appareil et compatibles avec le type de source lumineuse ou de guide de lumière utilisés.

Le dessus du boîtier 410 est renforcé par une nervures411.

La figure 10A est une vue de dessus de la cartouche 400 montrant sa fenêtre 431 derrière laquelle se trouve la source lumineuse commandée.

La vue de côté de la figure 10B montre tout particulièrement les nervures de guidage 460 et les zones de préhension 450.

Cette vue de côté laisse apparaître le plan de jonction des deux parties 430, 440 du boîtier qui est sensiblement parallèle au plan de l'emplacement de la cartouche dans la coque.

La vue de dessous de la figure 10C montre comme la figure 10A la forme globale de la cartouche.

Suivant une variante non représentée, la pièce à main comporte des cellules d'analyse de la pigmentation de la peau pour analyser la peau avant tout traitement, par mesure de sécurité. Cela permet également à l'opérateur de choisir les paramètres de traitement dans le sens d'une bonne sécurité et de l'efficacité du traitement.

La coque 110, son capot 120 ainsi que la cartouche 400 et l'embase 500 sont, de préférence, des pièces réalisées en matière plastique par injection.

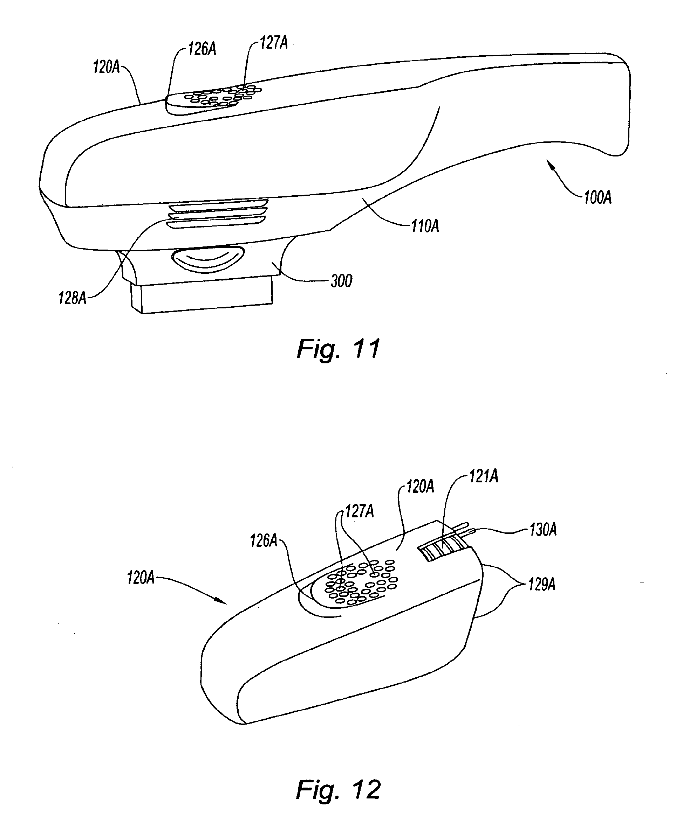

Les figures 11 à 14 montrent une variante de réalisation d'une pièce à main 100A. Pour sa description, on utilisera les mêmes références que pour le premier mode de réalisation en complétant ces références, le cas échéant, du suffixe A pour les modifications concernées par la variante.

Ainsi, selon la figure 11, la variante de pièce à main 100A se compose d'une coque 110A logeant de manière amovible, une cartouche et un guide de lumière 300 apparaissant en partie comme dépassant du dessous de la pièce à main. A l'emplacement de la cartouche 400, le dessus du capot 120A de la pièce à main comporte une partie 126A venant légèrement en saillie munie de plusieurs orifices de ventilation 127A et dans le prolongement de ces orifices d'aération selon la figure 11, les deux flancs de la coque 110A de la pièce à main, au-dessus du guide de lumière 300, il y a des fentes de ventilation 128A. Les orifices 127A sur le dessus et les fentes de ventilation 128A permettent de créer un passage d'air, forcé par un ventilateur 140A intégré à la pièce à main 100A.

La figure 12 montre de façon plus détaillée le capot 120A de la pièce à main 100A avec les orifices 127A d'entrée d'air sur le dessus et la partie renflée 126A destinée à absorber la surépaisseur occasionnée par le logement recevant le ventilateur.

Le bord arrière 128A du capot 120A comporte deux broches 130A pour l'alimentation électrique du ventilateur. Ces deux broches viennent s'engager dans des douilles de contact portées par la coque 110A au niveau de son ouverture recevant le capot 120A.

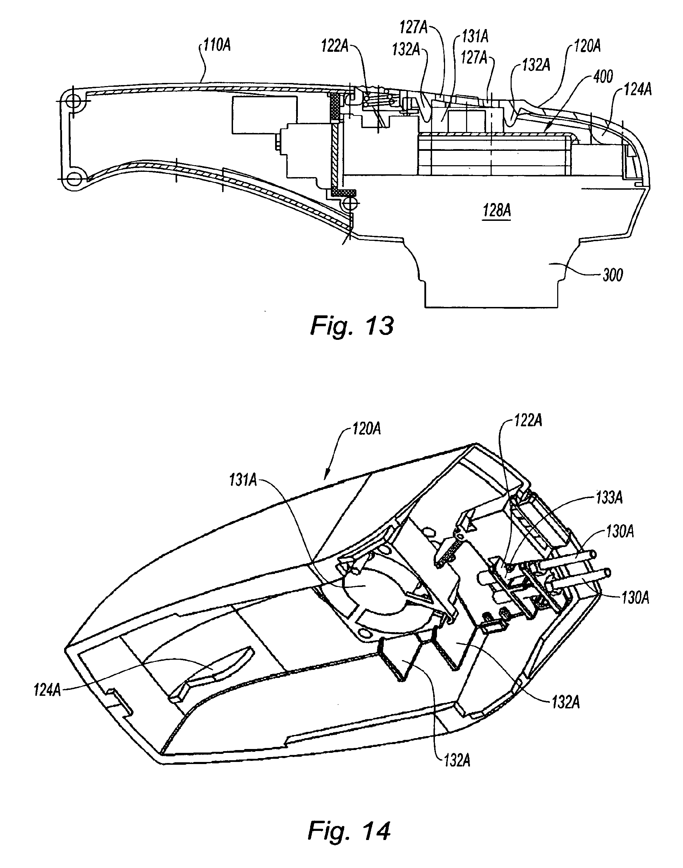

La vue en coupe longitudinale partielle de la figure 13 montre la position du ventilateur 131A à l'intérieur du capot 120A de la pièce à main. Le ventilateur est situé exactement au-dessus de la cartouche 400, au niveau de son milieu qui est l'endroit le plus chaud de la cartouche. Le ventilateur 131A est encastré ou enclipsé dans un logement défini par des languettes 132A moulées avec le capot. Le boîtier du ventilateur 131A est ainsi encastré dans le capot et y est retenu.

L'alimentation du ventilateur se fait par les deux broches 130A évoquées ci-dessus qui n'apparaissent pas dans cette figure 13.

La figure 13 montre également de manière esquissée les fentes de ventilation 128A sur les deux côtés de la coque 110A au niveau de la cartouche 400, légèrement en dessous de celle-ci de façon que les flux d'air aspiré par le ventilateur 131A à travers des orifices 127A dans le dessus du capot, au niveau de son emplacement, balaie également les côtés de la cartouche 400 pour sortir à travers les fentes 128A de la coque 110A.

La figure 14 montre de manière plus détaillée, l'installation du ventilateur 131A dans la paroi du dessus du capot 120A. Le ventilateur est fixé dans des cloisons formant les languettes 132A dans lesquelles il est enclipsé.

L'alimentation électrique du ventilateur se fait par l'intermédiaire des deux broches 130As, portées par un support 133A. Les broches sont reliées au ventilateur par des câbles non représentés. Elles sont destinées à s'engager dans des logements de contact portés par la coque 110A.

Les broches 130A sont placées à côté du crochet 122A qui verrouille le capot 120 à la coque 110A. Les autres éléments du capot 120A, identiques à ceux du premier mode de réalisation, portent les mêmes références complétées par le suffixe A et leur description ne sera pas reprise.

Le circuit de commande du ventilateur 131A n'a pas été représenté. Il assure l'alimentation appropriée du ventilateur 131A pour que celui-ci débite selon les besoins. Le circuit de commande comprend une sonde de température logée dans la coque 110A pour détecter la température de l'enceinte fermée, constituée par la coque 110A et le capot 120A autour de la cartouche 400. Le signal de température est comparé à une température limite, enregistrée. En fonction du résultat de la comparaison, le ventilateur 131A sera commandé à l'une des trois vitesses correspondant à trois débits différents. La commande du moteur de ventilateur 131A se fait par le réglage du niveau de tension alimentant le moteur. Enfin, il est prévu une sécurité pour une situation extrême pour laquelle il s'avérerait impossible de réduire la température en dessous d'un seuil fixé et, dans ce cas extrême, le fonctionnement de l'appareil serait arrêté.

### NOMENCLATURE

- 100, 100A: pièce à main
- 110, 110A: coque
- 110a, 110b: parties de la coque
- 111: organe d'accrochage
- 112: liaison vissée / vis / point de vissage
- 113: orifice renforcé
- 114: cheminée de vissage
- 115: glissière latérale
- 116: nervure
- 117: nervure
- 118: rails latéraux supplémentaires
- 119: bec
- 120, 120A: capot
- 121, 121A: poussoir
- 122, 122A: crochet
- 123: retour
- 124: butée
- 126A: partie en saillie
- 127A: orifices de ventilation
- 128A: fentes de ventilation
- 129A: bord arrière
- 130A: broches
- 131A: ventilateur
- 132A: languette
- 133A: support des broches

- 200: liaisons fluidiques électriques

- 300: guide de lumière
- 301: surface d'émission

- 400: cartouche
- 401: source lumineuse
- 402: canal de fluide caloporteur
- 410: base du boîtier
- 411: nervure de guidage
- 421: prise ou connecteur
- 422: embout avec des joints
- 430: partie inférieure
- 431: fenêtre
- 440: partie supérieure
- 450: zone de préhension
- 460: nervure de guidage

- 500: embase
- 510: plaque
- 511: orifice de vissage
- 520: branchements électroniques / électriques
- 530: ajutage

## Revendications

1. Pièce à main pour un appareil de phototraitement cosmétique, médical/thérapeutique de la peau, reliée à un poste d'alimentation en énergie et en fluide caloporteur de refroidissement par une liaison par câble et tubes dans l'appareil et, comprenant
- une coque (110) logeant, de manière amovible, une cartouche (400) à fenêtre (431) ayant une source lumineuse et son circuit de refroidissement, un capot (120) fermant l'emplacement de la cartouche dans la coque (110),
- un guide de lumière (300) relié à la source lumineuse par la fenêtre (431) de la cartouche (400) et dont la surface de sortie (301) est destinée à être appliquée contre ou au-dessus de la surface de la peau à traiter,
**caractérisée en ce que**
- l'emplacement de la cartouche (400) au-dessus du guide de lumière (300), est défini par une embase (500) portant les organes de connexion (520, 530) de la cartouche et par une surface de guidage dont une extrémité correspond à l'embase (500) et les côtés sont des rails latéraux de guidage (115) autour de l'emplacement du guide de lumière et
- la cartouche (400) est en forme de boîtier plat (410) dont une extrémité porte une plaque transversale d'assemblage (420) munie des organes de connexion (421, 422) homologues à ceux de l'embase (500) et les côtés comportent des nervures de guidage (460) pour coopérer avec les rails latéraux (115) de l'emplacement et positionner la cartouche (400) et la guider dans son mouvement de translation vers son emplacement pour le branchement et la connexion en fin de course et la maintenir connectée.

2. Pièce à main selon la revendication 1,
**caractérisée en ce qu'**
- à l'emplacement de la cartouche (400), la coque (110) comporte des rails supplémentaires (118) parallèles à ceux destinés à guider la cartouche (400), ainsi qu'un organe d'enclipsage (111),
- le capot (120) couvrant l'emplacement de la cartouche (400) et complétant la coque (110), comporte des organes de guidage (123) pour coulisser dans les rails supplémentaires (118), ainsi qu'un organe d'enclipsage complémentaire (111, 122) pour coopérer avec celui (112) de la coque (110) en position de fermeture du capot (120).

3. Pièce à main selon la revendication 1,
**caractérisée en ce que**
le boîtier (410) de la cartouche (400) comporte deux zones latérales de préhension (450) pour permettre de pincer le boîtier à la main ou le dégager et le mettre en place, par un mouvement de coulissement de ses nervures (460) dans les rails (115).

4. Pièce à main selon la revendication 1,
**caractérisée en ce que**
- le boîtier de la cartouche (400) est formé de deux parties (430, 440) logeant la source lumineuse et son circuit de fluide caloporteur de refroidissement,
- l'une (430) des parties a une fenêtre (431) dans laquelle apparaît la source lumineuse pour transmettre son flux lumineux,
- les deux parties étant assemblées selon un plan de jonction sensiblement parallèle au plan de l'emplacement,
- la partie inférieure (430) portant les nervures (460) et son extrémité, porte la plaque d'assemblage (420).

5. Pièce à main selon la revendication 1,
**caractérisée en ce que**
la coque (110) formant une poignée et une tête logeant la cartouche (400), est composée de deux parties (110a, b) se rejoignant sensiblement dans le plan médian (VI VI) et du capot (120) au-dessus de l'emplacement de la cartouche (400), ainsi que d'un logement pour recevoir l'embase (500) pour le branchement de la cartouche (400).

6. Pièce à main selon la revendication 1,
**caractérisée en ce que**
le capot (110A) comporte un ventilateur (121A) sous sa paroi supérieure munie d'orifices de ventilation (127A) et au-dessus de l'emplacement de la cartouche (400),
- la coque (110A) ayant dans ses parois latérales, de part et d'autre de la cartouche (400), des fentes de ventilation (128A) pour la sortie d'air,
- un chemin de passage d'air étant formé entre les orifices (127A) du dessus du capot (120A), à travers le ventilateur (121A) et le dessus de la cartouche (400) pour se diviser vers les côtés de la cartouche et sortir par les fentes de ventilation (128A) de la coque (110A) à côté de la cartouche (400).

7. Pièce à main selon la revendication 6,
**caractérisée en ce que**
le capot (120A) comporte deux broches (130A) d'alimentation électrique du moteur du ventilateur (131A) pour venir dans des douilles de la coque (110A) à côté du crochet de verrouillage (122).

8. Pièce à main selon la revendication 1,
**caractérisée par**
un circuit de commande pour le fonctionnement du ventilateur (vitesse de fonctionnement/débit) relié à une sonde de température associée à l'enceinte de la poignée [coque (110A) et capot (120)] pour commander le fonctionnement de ventilateur (131A) en fonction de la température détectée à l'intérieur de l'enceinte de la pièce à main (100A).

9. Pièce à main selon la revendication 6,
**caractérisée en ce que**
le boîtier de ventilateur (131A) est clipsé dans des languettes (132A) de retenue moulées en une seule pièce avec le capot (120A).

## Patentansprüche

1. Handstück für ein Gerät zur kosmetischen, medizinischen/therapeutischen Lichtbehandlung der Haut, das über eine Kabel- und Rohrverbindung im Gerät mit einer Einrichtung zur Versorgung mit Energie und Kühlfluid verbunden ist und Folgendes umfasst:
- einen Körper (110), in dem auf abnehmbare Weise eine Kartusche (400) mit Fenster (431) untergebracht ist, die eine Lichtquelle und ihren Kühlkreis umfasst, wobei eine Haube (120) die Einbaustelle der Kartusche im Körper (110) bedeckt,
- einen Lichtleiter (300), der über das Fenster (431) der Kartusche (400) mit der Lichtquelle verbunden ist und dessen Austrittfläche (301) dazu bestimmt ist, an oder über der zu behandelnden Hautoberfläche angebracht zu werden,
**dadurch gekennzeichnet, dass**
- die Einbaustelle der Kartusche (400) über dem Lichtleiter (300) durch einen Sockel (500), der die Anschlussorgane (520, 530) der Kartusche trägt, und durch eine Führungsfläche definiert wird, deren eines Ende dem Sockel (500) entspricht und deren Seiten seitliche Führungsschienen (115) um die Einbaustelle des Lichtleiters sind, und
- die Kartusche (400) die Form eines flachen Gehäuses (410) aufweist, dessen eines Ende eine quer angeordnete Verbindungsplatte (420) trägt, die mit Anschlussorganen (421, 422) versehen ist, die jenen des Sockels (500) entsprechen, und deren Seiten Führungsrippen (460) umfassen, um mit den seitlichen Schienen (115) der Einbaustelle zusammenzuwirken und um die Kartusche (400) zu positionieren und sie bei ihrer Verschiebebewegung zu ihrer Einbaustelle zu führen, so dass sie am Ende der Bewegung verbunden und angeschlossen wird und angeschlossen gehalten wird.

2. Handstück nach Anspruch 1,
**dadurch gekennzeichnet, dass**
- der Körper (110) an der Einbaustelle der Kartusche (400) zusätzliche Schienen (118), die parallel zu jenen sind, die zur Führung der Kartusche (400) bestimmt sind, sowie ein Schnappverbindungsorgan (111) umfasst,
- die Haube (120), welche die Einbaustelle der Kartusche (400) bedeckt und den Körper (110) vervollständigt, Folgendes umfasst: Führungsorgane (123), die in den zusätzlichen Schienen (118) gleiten, sowie ein zusätzliches Schnappverbindungsorgan (111, 122) das in der Verschlussposition der Haube (120) mit jenem (112) des Körpers (110) zusammenwirkt.

3. Handstück nach Anspruch 1,
**dadurch gekennzeichnet, dass**
das Gehäuse (410) der Kartusche (400) zwei seitliche Griffbereiche (450) umfasst, um das Gehäuse mit der Hand ergreifen zu können oder um es durch eine Gleitbewegung seiner Rippen (460) in den Schienen (115) herausziehen und wieder anbringen zu können.

4. Handstück nach Anspruch 1,
**dadurch gekennzeichnet, dass**
- das Gehäuse der Kartusche (400) aus zwei Teilen (430, 440) gebildet ist, welche die Lichtquelle und ihren Kühlfluidkreis aufnehmen,
- einer (430) der Teile ein Fenster (431) aufweist, in dem die Lichtquelle erscheint, um ihren Lichtstrom auszusenden,
- die zwei Teile gemäß einer Verbindungsebene miteinander verbunden sind, die im Wesentlichen parallel zur Ebene der Einbaustelle ist,
- der untere Teil (430) die Rippen (460) trägt und sein Ende die Verbindungsplatte (420) trägt.

5. Handstück nach Anspruch 1,
**dadurch gekennzeichnet, dass**
der Körper (110), der einen Griff und einen Kopf zur Aufnahme der Kartusche (400) bildet, aus Folgendem gebildet ist: aus zwei Teilen (110a, b), die im Wesentlichen in der Mittelebene (VI VI) miteinander verbunden sind, aus der Haube (120) über der Einbaustelle der Kartusche (400) sowie aus einem Fach zur Aufnahme des Sockels (500) zum Anschluss der Kartusche (400).

6. Handstück nach Anspruch 1,
**dadurch gekennzeichnet, dass**
die Haube (110A) unter ihrer oberen Wand, die mit Lüftungsöffnungen (127A) versehen ist, und über der Einbaustelle der Kartusche (400) einen Lüfter (121A) umfasst,
- der Körper (110A) in seinen Seitenwänden zu beiden Seiten der Kartusche (400) Lüftungsschlitze (128A) zum Austritt von Luft umfasst,
- ein durch den Lüfter (121A) verlaufender Luftdurchlassweg zwischen den Öffnungen (127A) an der Oberseite der Haube (120A) und der Oberseite der Kartusche (400) gebildet ist, der sich zu den Seiten der Kartusche hin teilt und durch die Lüftungsschlitze (128A) des Körpers (110A) an der Seite der Kartusche (400) austritt.

7. Handstück nach Anspruch 6,
**dadurch gekennzeichnet, dass**
die Haube (120A) zwei Stifte (130A) zur Stromversorgung des Motors des Lüfters (131A) umfasst, die in Buchsen des Körpers (110A) neben dem Verriegelungshaken (122) eingeführt werden.

8. Handstück nach Anspruch 1,
gekennzeichnet durch
einen Steuerkreis für den Betrieb des Lüfters (Betriebsgeschwindigkeit / Durchsatz) umfasst, der mit einer Temperatursonde verbunden ist, die mit der Ummantelung des Griffs [Körper (110A) und Haube (120)] verbunden ist, um den Betrieb des Lüfters (131A) in Abhängigkeit von der Temperatur zu steuern, die im Inneren der Ummantelung des Handstücks (100A) erkannt wurde.

9. Handstück nach Anspruch 6,
**dadurch gekennzeichnet, dass**
das Gehäuse des Lüfters (131A) in Schnappverbindung mit Haltelaschen (132A) gebracht wird, die einstückig mit der Haube (120A) geformt sind.

## Claims

1. Handpiece for a cosmetic, medical/therapeutic skin phototherapy device, which handpiece is connected to a power and liquid coolant supply unit by a cable and tube connection and comprises
- a body (110) which houses, in a removable manner, a cartridge (400) with a window (431) having a light source and its cooling circuit, a cover (120) closing the seat of the cartridge in the body (110),
- a light guide (300) which is connected to the light source by the window (431) of the cartridge (400) and the outlet surface (301) of which is to be applied to or above the surface of the skin to be treated,
**characterised in that**
- the seat of the cartridge (400) above the light guide (300) is defined by a base (500) carrying the connection members (520, 530) of the cartridge and by a guide surface, one end of which corresponds to the base (500) and the sides of which are lateral guide rails (115) around the seat of the light guide, and
- the cartridge (400) is in the form of a flat housing (410), one end of which carries a transverse assembly plate (420) which is provided with connection members (421, 422) homologous to those of the base (500) and the sides of which comprise guide ribs (460) for cooperating with the lateral rails (115) of the seat and positioning the cartridge (400) and guiding it in its movement of translation towards its seat for attachment and connection at the end of its travel, and for keeping it connected.

2. Handpiece according to claim 1,
**characterised in that**
- at the seat of the cartridge (400), the body (110) comprises additional rails (118) parallel to those which are to guide the cartridge (400), as well as a clip-fastening member (111),
- the cover (120) which covers the seat of the cartridge (400) and completes the body (110) comprises guide members (123) for sliding in the additional rails (118), as well as a complementary clip-fastening member (111, 122) for cooperating with the clip-fastening member (112) of the body (110) when the cover (120) is in the closed position.

3. Handpiece according to claim 1,
**characterised in that**
the housing (410) of the cartridge (400) comprises two lateral gripping regions (450) for allowing the housing to be gripped by hand or removed and fitted by a sliding movement of its ribs (460) in the rails (115).

4. Handpiece according to claim 1,
**characterised in that**
- the housing of the cartridge (400) is formed of two parts (430, 440) which house the light source and its liquid coolant circuit,
- one (430) of the parts has a window (431) in which the light source appears in order to transmit its luminous flux,
- the two parts being assembled according to a joining plane which is substantially parallel to the plane of the seat,
- the lower part (430) carrying the ribs (460) and its end carries the assembly plate (420).

5. Handpiece according to claim 1,
**characterised in that**
the body (110) forming a handle and a head which houses the cartridge (400) is composed of two parts (110a, b), which are joined substantially in the mid-plane (VI VI), and of the cover (120) above the seat of the cartridge (400) as well as of a housing for receiving the base (500) for the attachment of the cartridge (400).

6. Handpiece according to claim 1,
**characterised in that**
the cover (110A) comprises a fan (121A) beneath its upper wall provided with ventilation orifices (127A) and above the seat of the cartridge (400),
- the body (110A) having in its lateral walls, on either side of the cartridge (400), ventilation slots (128A) for the exit of air,
- an air passageway being formed between the orifices (127A) in the top of the cover (120A), through the fan (121A) and the top of the cartridge (400) and then dividing towards the sides of the cartridge and emerging through the ventilation slots (128A) of the body (110A) next to the cartridge (400).

7. Handpiece according to claim 6,
**characterised in that**
the cover (120A) comprises two pins (130A) for supplying power to the motor of the fan (131A), which pins engage in sockets in the body (110A) next to the locking hook (122).

8. Handpiece according to claim 1,
**characterised by**
a control circuit for the operation of the fan (operating speed/flow rate) which is connected to a temperature sensor associated with the enclosure of the handle [body (110A) and cover (120)] for controlling the operation of the fan (131A) according to the temperature detected inside the enclosure of the handpiece (100A).

9. Handpiece according to claim 6,
**characterised in that**
the housing of the fan (131A) is clipped into retaining tabs (132A) which are integrally moulded with the cover (120A).
